# EUROPÄISCHE PATENTSCHRIFT

(11) **EP 0 950 424 B1**
(45) Veröffentlichungstag und Bekanntmachung des Hinweises auf die Patenterteilung: **01.06.2005**
(21) Anmeldenummer: 99106488.2
(22) Anmeldetag: 30.03.1999
(51) Int. Cl.: A61M 25/00, F16L 11/08

(54) **Verfahren zur Herstellung armierter, medizinischer Schläuche**
Method of manufacture for reinforced medical tubings
Méthode de fabrication de tubes médicaux renforcés

(30) Priorität: 17.04.1998 DE 19816986
(43) Veröffentlichungstag der Anmeldung: 20.10.1999
(73) Patentinhaber: RAUMEDIC AG, 95233 Helmbrechts (DE)
(72) Erfinder: Ziembinski, Ralf Dr. c/o Rehau AG & Co, 95111 Rehau (DE); Koos, Norbert c/o Rehau AG & Co., 95111 Rehau (DE)
(74) Vertreter: Hofmann, Matthias, Dr.

(56) Entgegenhaltungen:
- EP-A- 0 361 314
- EP-A- 0 715 863
- WO-A-96/33763
- US-A- 3 805 848
- US-A- 4 676 229
- US-A- 5 647 400

## Beschreibung

Die Erfindung betrifft ein Verfahren zur Herstellung armierter, medizinischer Schläuche wie Herzkanülen, Trachealtuben, Tracheostomietuben und dergleichen, wobei die Armierung eine spiralförmige Verstärkung in Form eines Metalldrahtes oder eines Kunststoff-Filaments ist und in vorgegebenem Windungsabstand in die Schlauchwand eingebracht wird, und wobei als erstes ein Grundschlauch aus einem polymeren Werkstoff extrudiert wird, auf dessen erkaltete Oberfläche die Spirale aufgewickelt und danach eine äußere Schlauchschicht aus einem polymeren Werkstoff auf diese Kombination Grundschlauch - Spirale aufgebracht wird.

Ein gattungsgemäßer armierter Schlauch für medizinische Anwendungen ist aus der EU 0 451 996 B1 bekannt. Der dort beschriebene verstärkte, medizinische Schlauch wird nach einem Verfahren hergestellt, bei dem zunächst in kontinuierlicher Extrusion ein flexibler Basisschlauch erzeugt wird. Dieser Basisschlauch wird abgekühlt und danach wird in einem kontinuierlichen Arbeitsverfahren eine steife Plastikfilament-Schraubenwicklung um den gekühlten Schlauch aufgewickelt. In einem weiteren Verfahrensschritt wird danach eine äußere Schicht aus flexiblem Material auf den Basisschlauch und die Filament-Schraubenwicklung aufextrudiert.

Durch diese Verfahrensweise wird ein endgültiger, kontinuierlicher flexibler Schlauch gebildet, der wenigstens eine zwischen dem Basisschlauch und der äußeren Schicht eingelegte Filament-Schraubenwicklung umfaßt. Das benötigte Schlauchstück wird danach auf seine endgültige Länge abgeschnitten, wobei auch die Filament-Schraubenwicklung durchtrennt wird. Dabei wird wenigstens ein abgeschnittenes Filamentende an einer Stirnfläche eines distalen Endes des endgültigen Schlauchstückes freigelegt. Dieses freigelegte Filamentende wird mit einer dünnen Materialschicht am distalen Ende derart abgedeckt, daß das abgeschnittene Filamentende nicht mehr freiliegt.

Die bereits genannte EP 0 451 996 B1 enthält umfangreiche Angaben zum hier gegebenen Stand der Technik, die im einzelnen nicht wiederholt werden sollen.

Es bleibt lediglich darauf hinzuweisen, dass nach dem Stand der Technik derartige Verstärkungen in Draht- oder Filamentform hinreichend bekannt sind. Derartige Verstärkungen sind im medizintechnischen Bereich der Anwendung dieser Schläuche erforderlich, um die Verbesserung der Knickbeständigkeit herbeizuführen und zu verhindern, dass die Schlauchwand während des Gebrauchs kollabiert.

Derartige Schläuche können als Katheter mit oder ohne Ballon eingesetzt werden, der zusätzlich zur Fixierung des distalen Katheterendes im Körper eines Patienten dienen kann bzw. dazu dient, einen Zugang im Körper des Patienten zu verschließen.

Bei den meisten Arten von medizintechnischen Schläuchen ist es wünschenswert, dass sie einerseits so flexibel wie möglich, andererseits aber in ihrer Wanddicke so gering wie möglich sind. Bei einer Verringerung der Wanddicke zur Erzielung einer größeren Flexibilität des Schlauches besteht aber immer auch die große Gefahr des Abknickens und damit auch des Kollabierens beim Gebrauch. Da das Abknicken bzw. Kollabieren solcher Schläuche zu einem vollständigen Verschluss des Schlauchlumens mit gravierenden Folgen für den betroffenen Patienten bis hin zu dessen Tod führen kann, muss der Schlauch so konstruiert sein, dass ein Abknicken bzw. Kollabieren bei der jeweiligen Anwendung ausgeschlossen ist. So ist in dem Britischen Patent 204 32 01 die Erhöhung der Knickbeständigkeit eines Schlauches mittels einer Faserverstärkung beschrieben. In dem Kanadischen Patent 1,199,761 wird vorgeschlagen, eine Helix aus einem Metalldraht oder einem synthetischen Filament als Verstärkung in die Schlauchwand einzubetten.

Eine Verstärkung durch einen Metalldraht hat jedoch den Nachteil eines ungenügenden Rückstellverhaltens. Zwar wird die Knickbeständigkeit im Vergleich zu einem nichtarmierten Schlauch bei vergleichbarer Wanddicke deutlich verbessert, jedoch bleibt der Schlauch dauerhaft deformiert, wenn er über die Bewegungsmöglichkeiten der Spirale hinaus abgeknickt wird. Nach einem Abknicken bzw. Kollabieren kann ein solcher metallspiralverstärkter Schlauch nicht mehr rückgestellt werden.

Mit synthetischen Filamenten spiralförmigverstärkte Schläuche bieten zusätzlich zu der verbesserten Knickbeständigkeit auch ein gutes Rückstellverhalten nach einem möglichen Abknicken bzw. Kollabieren des Schlauches. Daher wird im Stand der Technik vorgeschlagen, ein synthetisches Filament zu verwenden, welches einen Abstand der spiralförmigen Windungen von wenigstens 0,5 mm aufweist.

Zur Herstellung eines solchen spiralförmig armierten Schlauches gibt es grundsätzlich zwei verschiedene Verfahren.

Zunächst besteht die Möglichkeit, auf einen Grundschlauch, der die innere Materialschicht des endgültigen Produktes darstellt, eine Metallfeder aufzuwickeln, aufzuschieben oder sonstwie anzuordnen und diese dann mit einer zweiten Materialschicht in der Schlauchwand einzubetten. Diese zweite Materialschicht kann im Wege der Koextrusion, aber auch über die Tauchtechnik aufgebracht werden. Alternativ zur Tauchtechnik lässt sich ein derartiger Schlauch aber auch durch ein On-Line-Koextrusionsverfahren herstellen, indem auf die innere Materialschicht der Metalldraht oder das synthetische Filament spiralfömig aufgebracht und durch eine zweite extrudierte Materialschicht eingebettet wird.

In jedem Fall muss dieser Schlauch dann noch zu einem gebrauchsfertigen Katheter konfektioniert werden. Dazu muss eine Katheterspitze am distalen Ende ankonfektioniert werden und ein Konnektor zu einem extracorporalen System am proximalen Ende angebracht werden. Unabhängig davon, mit welcher Verbindungstechnik diese Teile an den Schlauch befestigt werden, besteht das Problem, dass am Schlauchende herausragender Metalldraht bzw. Monofilament seitlich aus dem fertigen Katheterprodukt hervorsteht. Dadurch entsteht eine Verletzungsgefahr sowohl beim Patienten als auch beim Anwender selbst, die nicht akzeptabel ist. Nach dem Stand der Technik wird diese Verletzungsgefahr dadurch vermieden, dass in aufwendiger Technik ein Verschluss im Bereich der Schneidkante des Schlauches angeformt wird, mit dem die sperrigen, über den Umfang des Schlauches hinausstehenden Enden der Metalldraht- bzw. Filamentwicklung abgedeckt werden.

Aus der US 4,676,229 A ist ein armierter, medizinischer Schlauch bekannt. Dieser weist eine Klebeschicht zwischen einer Armierungswendel und einem Grundschlauch auf. Der für die Armierungswendel eingesetzte Draht hat einen Durchmesser zwischen etwa 0,01 und etwa 0,04 mm (0,004 bis 0,016 Zoll) und eine zwei- bis dreieinhalbmal so große Steigung. Der Schlauchinnendurchmesser liegt zwischen 3 und 4,5 mm.

Die WO 96/33763 A2 beschreibt metallische Armierungsbänder mit rechteckigem, ovalem oder halbovalem Querschnitt.

Aus der US 3,805,848 A ist ein für Kühlsysteme einsetzbarer armierter Schlauch beschrieben, bei dem eine Armierungsschicht zwischen einem Innen- und einem Außenschlauch verklebt ist.

Die EP 0 361 314 A2 beschreibt Ausführungsvarianten medizinischer Schläuche mit einer Kevlar-Faserschicht, die eingebettet ist zwischen einer Innenlage aus Teflon und einer Außenhülle aus Polyethylen. Die Teflon-Innenlage umgibt ein großes, inneres Lumen. Ein zusätzliches Lumen, welches zur Führung einer Steuerleitung dient, ist zwischen der Teflon-Innenlage und der Kevlar-Faserschicht angeordnet.

Die Erfindung hat es sich zur Aufgabe gestellt, den Nachteil des hervorstehenden Metalldraht- bzw. Monofilamentendes zu vermeiden und ein Verfahren anzugeben, mit dem die Armierung unlösbar in der Schlauchwand fixiert wird. Gleichzeitig soll der Schlauch gegen ein Abknicken stabilisiert sein. Erfindungsgemäß wird dazu vorgeschlagen, dass der äußere Umfang des Grundschlauches vor dem Aufwickeln der Spirale mit einem Klebemittel fortlaufend beschichtet wird. Dieses Klebemittel kann ein Lösungsmittel oder ein herkömmlicher Schmelzkleber sein. Erfindungsgemäß verwendet werden Drähte oder Filamente mit einer Dicke von wenigstens 0,05 mm und höchstens 2 mm. Die Breite der Drähte bzw. Filamente beträgt wenigstens 0,15 mm bei einem Schlauchinnendurchmesser, der wenigstens 2 mm, höchstens aber 30 mm beträgt. Das zusätzliche Lumen befindet sich in einem aufgesetzten Teil des Schlauchprofils, was zu einer insgesamt ovalen Form des Schlauchquerschnitts führt. Dadurch wird einerseits eine geringe Wanddicke des Schlauches ermöglicht und eine zusätzliche Stabilisierung gegen das Abknicken durch die Wandverdickung um das kleine Lumen herbeigeführt.

Bei der Verwendung einer Spirale aus Metalldraht ist erfindungsgemäß deren Umfang mit einer mit dem Klebemittel des Grundschlauchumfangs verträglichen Beschichtung versehen. Ist die Spirale dagegen ein Kunststoff-Filament, dann kann dieses aus einem mit dem Klebemittel des Grundschlauchumfangs verträglichen Polymer bestehen.

Da die beiden Teilelemente Grundschlauchumfang - Spirale auf diese Weise eine unlösbare Verbindung eingehen, ergibt sich eine zusätzliche Verbesserung der Knickstabilität eines derart aufgebauten Schlauches. Diese Verbesserung ist nach dem Stand der Technik nicht möglich, da dort eine haftende Verbindung zwischen der Spirale und dem Schlauch nicht gegeben ist, was sich durch den Nachteil manifestiert, dass nach dem Schneiden des Schlauches auf Endlänge die Spitzen der Spirale in der als nachträglich geschilderten Weise an der Schneidkante austreten.

In einem vorgegebenen Beispielsfall kann das Schlauchmaterial des Grundschlauchs aus einem weichmacherhaltigen Polyvinylchlorid mit einem Weichmachergehalt von wenigstens 10 % bestehen. Als Weichmacher kann herkömmliches DEHP eingesetzt werden; es sind jedoch auch andere Weichmacher wie z.B. TEHTM einsetzbar.

Das Filament kann im vorgegebenen Beispielsfall aus Hart-PVC bestehen, also einer weichmacherfreien PVC-Type und als Klebemittel zwischen beiden Elementen kann Cyclohexanon, Tetrahydrofuran oder Methylethylketon jeweils als Lösemittelkleber bzw. Acrylate eingesetzt werden.

Wenn es erforderlich ist, dass die Position des Katheterschlauches im Röntgenbild sichtbar gemacht werden muss, dann ist diese Forderung bei der Verwendung einer Armierung mit einer Metallspirale durch den Röntgenkontrast des Metalls bereits gewährleistet.

Ein synthetisches Filament weist jedoch keine Röntgenkontrastfähigkeit auf. Es wird daher im Rahmen der Erfindung als vorteilhaft angesehen, ein solches synthetisches Filament aus einem polymeren Werkstoff herzustellen, der einen ausreichenden Gehalt einer röntgenkontrastfähigen Substanz enthält. Als eine solche Substanz kann beispielsweise Bariumsulfat eingesetzt werden und um einen ausreichenden Röntgenkontraststreifen sicherzustellen, muß die Zumischung zum Grundpolymer wenigstens 10 Gewichtsprozent Bariumsulfat oder eines anderen röntgenkontrastfähigen Materials, z. B. eines Schwermetallpulvers, betragen. Beim Einsatz eines solchen, mit Bariumsulfat gefüllten synthetischen Filaments erübrigt sich die Einextrusion eines Streifens aus einem röntgenkontrasthaltigen Material in die Schlauchwand.

Erfindungsgemäß kann der Querschnitt der Verstärkungsspirale aus einem herkömmlichen Runddraht bzw. einem Rundfilament bestehen. Es kann jedoch auch ein Flachdraht bzw. ein Flachfilament verwendet werden, um dadurch die Wanddicke des Schlauches möglichst gering zu halten. Hier kommt der Umstand zum Tragen, daß die Verbesserung der Knickstabilität im wesentlichen von der Querschnittsfläche der verwendeten Armierung abhängt und nicht von deren Raumform. Erfindungsgemäß verwendet werden können Drähte oder Filamente mit einer Dicke von wenigstens 0,05 mm und höchstens 2 mm. Die Breite der Drähte bzw. Filamente sollte wenigstens 0,15 mm betragen bei einem Schlauchinnendurchmesser, der wenigstens 2 mm, höchstens aber 30 mm beträgt.

Wird der Schlauch als Halbzeug für einen Trachealtubus oder einen Tracheostomietubus eingesetzt, so muß die Schlauchwand ein kleines Lumen als Zugang zu dem am distalen Ende des Katheters befestigten Ballon aufweisen. Die Erfindung sieht für diesen Fall vor, das Lumen mit einem Durchmesser von wenigstens 0,3 mm in einer weiteren, dritten Materialschicht anzuordnen. Das Lumen soll sich dabei in einem aufgesetzten Teil des Schlauchprofils befinden, was zu einer insgesamt ovalen Form des Schlauchquerschnitts führt. Dadurch wird einerseits eine geringe Wanddicke des Schlauches ermöglicht und eine zusätzliche Stabilisierung gegen das Abknicken durch die Wandverdickung um das kleine Lumen herbeigeführt.

In der Zeichnung sind Ausführungsbeispiele eines mit dem erfindungsgemäßen Verfahren hergestellten Schlauches schematisch dargestellt; es zeigt:
- Figur 1: einen Querschnitt durch einen zweischichtigen Schlauchaufbau
- Figur 2: die Seitenansicht eines Schlauchstückes mit angedeuteter Spiralamierung
- Figur 3: den Querschnitt durch einen dreischichtigen Schlauchaufbau mit Zusatzlumen

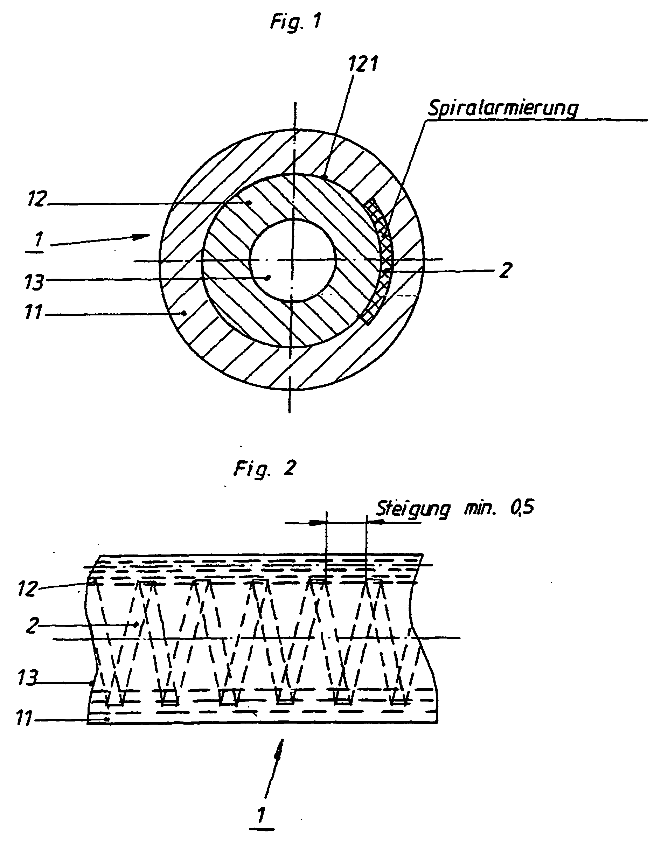

Figur 1 zeigt den Querschnitt durch den Schlauch 1 mit den Schlauchschichten 11, 12 und dem Lumen 13. Auf den äußeren Umfang 121 des Grundschlauches 12 ist die Spirale 2 nach dem Verfahren der Erfindung fest und unverrückbar aufgebracht. Die in der Figur dargestellte Spirale 2 ist als rechteckiges Filament ausgebildet. Der Grundschlauch 12 bildet zusammen mit dem Deckschlauch 11 und der Spirale 2 aufgrund der gegebenen Beschichtungs- bzw. Materialkonditionen ein fest miteinanderverklebtes bzw. verschweißtes Gebilde, dessen Einzelteile auch beim Schneiden nicht voneinander getrennt werden können.

Figur 2 zeigt die Seitenansicht eines Schlauchstücks des Aufbaues aus Figur 1 mit der angedeuteten Spirale 2, deren Steigung wenigstens 0,5 mm beträgt.

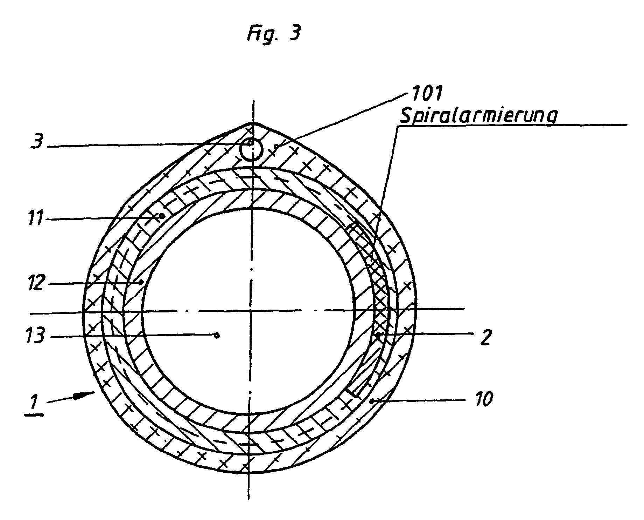

Figur 3 zeigt den Querschnitt durch einen dreischichtigen Schlauch 1 mit dem Lumen 13, dem Grundschlauch 12, der mittleren Schlauchschicht 11 und dem Deckschlauch 10. In die mittlere Schlauchschicht 11 ist die Spirale 2 der Armierung eingesetzt. Die Deckschicht 10 besitzt eine im oberen Scheitelpunkt angeordnete Materialverdickung 101, in welcher das kleine Lumen 3 durchgehend angeordnet ist. Die Materialverdickung 101 bewirkt, daß das Profil des Schlauches 1 in dieser Ausführungsform eine insgesamt ovale Form in seinem Querschnitt aufweist. Diese Form dient zur weiteren Stabilisierung des Schlauches 1 und damit zur Erhöhung der Knickbeständigkeit.

## Patentansprüche

1. Verfahren zur Herstellung armierter, medizinischer Schläuche, wobei die Armierung eine spiralförmige Verstärkung in Form eines Metalldrahtes oder eines Kunststofffilamentes ist und im vorgegebenen Bindungsabstand in die Schlauchwand eingebracht wird, und wobei als erstes ein Grundschlauch aus einem polymeren Werkstoff extrudiert wird, auf dessen erkaltete Oberfläche die Spirale aufgewickelt und danach eine zweite Schlauchschicht aus einem polymeren Werkstoff auf diese Kombination Grundschlauch - Spirale aufgebracht wird,
**dadurch gekennzeichnet, dass**
a) der äußere Umfang des Grundschlauches vor dem Aufwickeln der Spirale mit einem Klebemittel fortlaufend beschichtet wird;
b) die Drähte oder die Filamente verfügen über
- eine Dicke von wenigstens 0,05 und höchstens 2 mm,
- eine Breite von wenigstens 0,15 mm,
- eine Steigung von wenigstens 0,5 mm;
c) der Schlauchinnendurchmesser wenigstens 2 mm, höchstens aber 30 mm beträgt;
d) eine dritte Schlauchschicht mit einer Wandverdickung auf die Kombination Grundschlauch - Spirale - zweite Schlauchschicht aufgebracht wird;
e) ein zusätzliches Lumen durchgehend in die Wandverdickung der dritten Schlauchschicht eingebracht wird.

2. Verfahren nach Anspruch 1, **dadurch gekennzeichnet, dass** die Spirale eine Metallspirale ist, deren Umfang eine mit dem Klebemittel des Grundschlauchumfangs verträgliche Beschichtung aufweist.

3. Verfahren nach Anspruch 1, **dadurch gekennzeichnet, dass** die Spirale aus einem Kunststofffilament besteht, welches aus einem mit dem Klebemittel des Grundschlauchumfangs verträglichen Polymeren besteht.

4. Verfahren nach Anspruch 3, **dadurch gekennzeichnet, dass** dem Polymeren des Kunststofffilaments eine röntgenkontrastfähige Substanz beigemengt ist.

5. Verfahren nach Anspruch 1, **dadurch gekennzeichnet, dass** der Draht oder das Filament der Spirale einen runden, flachen oder rechteckigen Querschnitt besitzt.

## Claims

1. Process to manufacture reinforced medical tubes, in which the reinforcement is a spiral brace in the form of a metal wire or plastic filament inserted at a specified pitch in the tube wall, wherein a basic tube is firstly extruded from a polymer material, on whose cooled surface the spiral is then coiled and then a second tube layer of a polymer material is applied to this combination of basic tube and spiral,
**characterised by**:
a) the outer perimeter of the basic tube is continuously coated with an adhesive before the spiral is coiled on;
b) the wires or filaments have
- a thickness of at least 0.05 mm and not more than 2 mm,
- a width of at least 0.15 mm,
- a pitch of at least 0.5 mm;
c) the inside diameter of the tube is at least 2 mm and not more than 30 mm;
d) a third tube layer with a thickened wall is applied to the combination of basic tube, spiral and second tube layer;
e) an additional lumen passing through the thickened wall of the third tube layer is inserted.

2. Process according to Claim 1, **characterised in that** the spiral is a metal spiral whose perimeter is coated with a coating compatible with the adhesive on the outer perimeter of the basic tube.

3. Process according to Claim 1, **characterised in that** the spiral consists of a plastic filament consisting of a polymer compatible with the adhesive on the outer perimeter of the basic tube.

4. Process according to Claim 3, **characterised in that** a substance capable of X-ray contrast is mixed with the polymer of the plastic filament.

5. Process according to Claim 1, **characterised in that** the wire or the filament of the spiral has a round, flat or rectangular cross-section.

## Revendications

1. Procédé pour la fabrication de tubes médicaux à armature, l'armature étant constituée d'un renforcement en spirale sous la forme d'un fil métallique ou d'un filament en matière plastique et intégrée avec un écart de spires donné dans la paroi du tube ; un tube de base est extrudé en premier d'une matière polymère, on enroule sur sa surface refroidie la spirale et on applique ensuite une deuxième couche tubulaire en matière polymère sur cette combinaison tube de base - spirale,
**caractérisé en ce que**:
a) le pourtour extérieur du tube de base est préparé sur toute la surface avec une colle avant l'enroulement de la spirale;
b) les fils métalliques ou filaments disposent
- d'une épaisseur d'au moins 0,05 et d'au plus 2 mm,
- d'une largeur d'au moins 0,15 mm,
- d'un pas d'au moins 0,5 mm;
c) le diamètre intérieur du tube mesure au moins 2 mm, mais au plus 30 mm;
d) une troisième couche tubulaire avec un épaississement de la paroi est appliquée sur la combinaison tube de base - spirale - deuxième couche tubulaire.
e) un lumen supplémentaire est inséré de bout en bout dans l'épaississement de la paroi de la troisième couche tubulaire

2. Procédé selon la revendication 1, **caractérisé en ce que** la spirale est une spirale métallique dont le pourtour présente un revêtement compatible avec la colle du pourtour du tube de base

3. Procédé selon la revendication 1, **caractérisé en ce que** la spirale est en filament de matière plastique, laquelle est en polymère compatible avec la colle du pourtour du tube de base.

4. Procédé selon la revendication 3, **caractérisé en ce qu'**une substance contrastant aux rayons X est mélangée au polymère du filament en matière plastique.

5. Procédé selon la revendication 1, **caractérisé en ce que** le fil métallique ou le filament de la spirale possède une coupe transversale ronde, plate ou rectangulaire.
